# EUROPÄISCHE PATENTANMELDUNG

(11) **EP 2 746 400 A1**
(43) Veröffentlichungstag der Anmeldung: **25.06.2014**
(21) Anmeldenummer: 12199048.5
(22) Anmeldetag: 21.12.2012
(51) Int. Cl.: C12P 13/00, C12N 15/52, C12N 9/00, C12N 9/10, C12N 9/12

(54) **Herstellung von Aminen und Diaminen aus einer Carbonsäure oder Dicarbonsäure oder eines Monoesters davon**

(71) Anmelder: Evonik Industries AG, 45128 Essen (DE)
(72) Erfinder: Schaffer, Steffen, 45699 Herten (DE); Gielen, Jasmin, 44879 Bochum (DE); Wessel, Mirja, 44799 Bochum (DE); Hennemann, Hans-Georg, 45770 Marl (DE); Häger, Harald, 59348 Lüdinghausen (DE); Volland, Michael, 48249 Dülmen (DE); Roos, Martin, 45721 Haltern am See (DE)

(57) **Zusammenfassung**

Die Erfindung betrifft einen Ganzzellkatalysator, der eine rekombinante α-Dioxygenase oder die Kombination aus einer rekombinanten Fettsäurereduktase und einer die Fettsäurereduktase phosphopantheteinylierenden Phosphopantheteinyl-Transferase exprimiert, und der zusätzlich zu der α-Dioxygenase und/oder der Kombination aus Fettsäurereduktase und Phosphopantheteinyl-Transferase eine Transaminase exprimiert, wobei die Phosphopantheteinyl-Transferase und/oder Transaminase bevorzugt rekombinant ist; sowie ein Verfahren zur Umsetzung einer Carbonsäure oder Dicarbonsäure oder eines Monoesters davon zu einem Amin oder Diamin, umfassend die Schritte Kontaktieren der Carbonsäure oder Dicarbonsäure oder des Monoesters davon mit einer phosphopantheteinylierten Fettsäurereduktase oder einer α-Dioxygenase und Kontaktieren des Produktes mit einer Transaminase.

## Beschreibung

Die Erfindung betrifft einen Ganzzellkatalysator, der eine rekombinante α-Dioxygenase und/oder die Kombination aus einer rekombinanten Fettsäurereduktase und aus einer die Fettsäurereduktase phosphopantheteinylierenden Phosphopantheteinyl-Transferase exprimiert, und der zusätzlich eine Transaminase exprimiert, wobei die Phosphopantheteinyl-Transferase und/oder Transaminase bevorzugt rekombinant ist; sowie ein Verfahren zur Umsetzung einer Carbonsäure oder Dicarbonsäure oder eines Monoesters davon zu einem Amin oder Diamin, umfassend die Schritte Kontaktieren der Carbonsäure oder Dicarbonsäure oder des Monoesters davon mit einer phosphopantheteinylierten Fettsäurereduktase oder einer α-Dioxygenase und Kontaktieren des Produktes mit einer Transaminase.

Polyamide sind eine Klasse von Polymeren, die durch sich wiederholende Amidgruppen gekennzeichnet sind. Der Begriff "Polyamid" betrifft im Unterschied zu den chemisch verwandten Proteinen gewöhnlich synthetische, im Handel erhältliche, thermoplastische Kunststoffe. Polyamide werden von primären Aminen oder von sekundären Aminen abgeleitet, die herkömmlich über gecrackte Kohlenwasserstoffe gewonnen werden. Es können jedoch auch Derivate, genauer Aminocarbonsäuren, Lactame und Diamine, zur Polymerherstellung verwendet werden. Von Interesse sind weiterhin kurzkettige, gasförmige Alkane als Edukte, die ausgehend von nachwachsenden Rohstoffen mit biotechnologischen Verfahren gewonnen werden können.

Die konventionelle chemisch-technische Erzeugung von derartigen Aminen und Diaminen ist von der Versorgung mit fossilen Rohstoffen abhängig, ineffizient, und es fallen dabei große Mengen unerwünschter Nebenprodukte an, in manchen Schritten der Synthese bis zu 80 %. Ein Beispiel für einen solchen Prozess stellt die Herstellung von Laurinlactam dar. Konventionell erfolgt diese über ein vielstufiges Verfahren, das nicht nur eine geringe Ausbeute ergibt, sondern gleichzeitig die Bereitstellung einer aufwändigen Infrastruktur erfordert.

Angesichts dieser Nachteile wurden Verfahren entwickelt, um Amine und Diamine unter Verwendung von Biokatalysatoren ausgehend von nachwachsenden Rohstoffen zu gewinnen. Als nachwachsende Rohstoffe kommen insbesondere Quellen für Fettsäuren in Frage, die in Form von Rapsöl, Kugeldistelöl, Palmkernöl, Kokosnussöl, Sonnenblumenkernöl und ähnlichen Naturprodukten aus einer Vielzahl von biologischen Quellen, insbesondere aus Pflanzen, gewonnen werden können.

PCT/EP 2008/067447 beschreibt ein biotechnologisches System zur Herstellung chemisch verwandter Produkte, genauer ω-Aminocarbonsäuren, unter Verwendung einer Zelle, die eine Reihe von geeigneten enzymatischen Aktivitäten aufweist und in der Lage ist, Carbonsäuren zu entsprechenden ω-Aminocarbonsäure umzuwandeln. Das Verfahren umfasst eine Kaskade enzymatisch katalysierter Reaktionen, insbesondere die Oxidation einer Fettsäure am endständigen Kohlenstoffatom bis zum Aldehyd und die anschließende Aminierung unter Verwendung einer Transaminase und einer Aminosäure als Amindonor, die über eine Aminosäuredehydrogenase regeneriert werden kann.

Ein bekannter Nachteil des dabei verwendeten AlkBGT-Oxidasesystems aus *Pseudomonas putida* GPO1 besteht jedoch darin, dass es nicht in der Lage ist, eine selektive Oxidation von aliphatischen Alkanen zu primären Alkoholen zu leisten. Vielmehr treten eine Vielzahl von Oxidationsprodukten auf; insbesondere erhöht sich der Anteil an höher oxidierten Produkten wie dem entsprechenden Aldehyd, Keton oder der entsprechenden Carbonsäure mit zunehmender Reaktionsdauer (C. Grant, J. M. Woodley and F. Baganz (2011), Enzyme and Microbial Technology 48, 480-486), was die Ausbeute an erwünschtem Amin entsprechend verringert.

Das Problem der relativ unselektiven Oxidation ist dadurch verschärft, dass die entstehenden Oxidationsprodukte strukturell sehr ähnlich sind. Dies bedingt, dass es sehr schwierig ist, sie von den erwünschten Oxidationsprodukten effizient und ohne signifikanten Ausbeuteverlust abzutrennen.

Ein weiterer Nachteil dieses Verfahrens besteht darin, dass überoxidierte Nebenprodukte, beispielsweise die Dicarbonsäure der als Edukt eingesetzten Fettsäure, das Recycling von hydrophoben Lösungsmitteln und hydrophoben flüssigen Kationenaustauschern, die gemäß der PCT/EP2011/071491 zur Abtrennung des Produktes aus der wässrigen Reaktionsmischung verwendet werden können, auf Kosten der Effizienz bei der Ressourcennutzung gehen.

In diesem Zusammenhang ist hervorzuheben, dass die Komplexität biotechnologischer Systeme mit einer Kaskade von Reaktionen wie dem in der PCT/EP 2008/067447 beschriebenen, von denen jeweils eine Reaktion von einem bestimmten Enzym katalysiert wird, die Optimierung der Reaktionsbedingungen erschwert. So ist im Falle der grundsätzlich reaktiven ω-Aminofettsäuren als Produkt die Möglichkeit gegeben, dass sie ab einer bestimmten kritischen Konzentration im Inneren der Zelle mit essentiellen Bestandteilen des Organismus reagieren und somit toxisch wirken. Ist das der Fall, so ist die Wachstums- und Synthesefähigkeit des Organismus bis hin zum Absterben der Zelle beeinträchtigt, ohne dass der Entwickler die Toxizität unmittelbar erkennen oder gar einem bestimmten Edukt, Zwischenprodukt oder Produkt zuordnen könnte. Welcher Organismus welche Konzentration an einem chemisch reaktiven Stoff verträgt, ist ebenfalls nicht vorhersehbar.

Auch mit Hinblick auf eine zu verbessernde Produktausbeute und eine zu verringernde Entstehung von Nebenprodukten kann der Fachmann limitierende und entscheidende Faktoren in einem System wie dem in der PCT/EP2008/067447 beschriebenen nicht routinemäßig identifizieren. Ist die Ausbeute an Produkt zu niedrig, so kann dies daran liegen, dass eines der Enzyme in einer zu niedrigen Konzentration vorhanden ist, ohne dass bekannt wäre, um welches der in Frage kommenden Enzyme es sich dabei handelt, d. h. das Edukt wird im vorgesehenen Zeitrahmen oder vor dem Abbau durch konkurrierende Enzyme aufgrund unzureichender Synthesekapazität nicht umgesetzt. Alternativ ist es durchaus möglich, dass ein Enzym zwar nachweisbar in der Zelle in Form eines Polypeptids vorhanden ist, aber gerade in dieser Zelle nicht die für die Aktivität essentielle Faltung aufweist oder ein bis dato unbekannter, für die Aktivität aber essentieller Cofaktor fehlt. Gleichermaßen kann, wie bereits erwähnt, das Stoffwechselprodukt für die Zelle toxisch sein oder abgebaut werden. Schließlich ist mit interferierenden Wechselwirkungen mit endogenen, d. h. natürlich in einer als Ganzzellkatalysator verwendeten Zelle vorhandenen Enzymen zu rechnen.

Es besteht somit Bedarf an Verfahren zur Herstellung von Alkylmonoaminen und -diaminen aus Fettsäuren, bei denen die enzymatisch katalysierten Reaktionen selektiver verlaufen und die Bildung unerwünschter Nebenprodukte minimiert ist.

Vor diesem Hintergrund besteht die der Erfindung zu Grunde liegende Aufgabe darin, ein mit Hinblick auf Ausbeute, Kohlenstoff- und/oder Stickstoffbilanz und/oder Reinheit möglichst effizientes biotechnologisches Verfahren zur Herstellung von Alkylmonoaminen und -diaminen aus Fettsäuren bereitzustellen.

Eine weitere der Erfindung zu Grunde liegende Aufgabe besteht darin, ein mit Hinblick auf Ausbeute, Kohlenstoff- und/oder Stickstoffbilanz, Wiederverwendbarkeit verwendeter Agenzien und/oder Reinheit des Produktes möglichst effizientes biotechnologisches Verfahren zur Herstellung von Alkylmonoaminen und -diaminen aus Fettsäuren bereitzustellen. In diesem Zusammenhang wird unter einer effizienten Kohlenstoff- und/oder Stickstoffbilanz bevorzugt verstanden, dass sich ein möglichst hoher Anteil des zur Umsetzung einer Carbonsäure oder eines Carbonsäureesters in Form von geeigneten Substraten an eine Zelle verfütterten Kohlenstoffs und/oder Stickstoffs im erwünschten Endprodukt wiederfindet, anstatt beispielsweise zu anderen Produkten als den erwünschten umgesetzt zu werden.

Eine weitere der Erfindung zu Grunde liegende Aufgabe besteht darin, die Aufarbeitbarkeit einer mehrphasigen Reaktionsmischung aus der Herstellung von Alkylmonoaminen und - diaminen aus Fettsäuren zu verbessern, besonders mit Hinblick auf die Wiederverwendbarkeit zur Aufarbeitung verwendeter hydrophober Lösungsmittel und flüssiger Kationenaustauscher, sowie mit Hinblick auf die Phasenbildung und -trennung bei einem biphasischen System umfassend eine wässrige Phase, in der die Umsetzung der Carbonsäure oder des Carbonsäureesters abläuft, und eine organische Phase mit organischen Lösungsmitteln und/oder flüssigen Kationenaustauschern.

Diese und weitere Aufgaben werden durch den Gegenstand der vorliegenden Anmeldung und insbesondere auch durch den Gegenstand der beigefügten unabhängigen Ansprüche gelöst, wobei sich Ausführungsformen aus den Unteransprüchen ergeben.

Das der Erfindung zu Grunde liegende Problem wird in einem ersten Aspekt gelöst durch einen Ganzzellkatalysator, der eine rekombinante α-Dioxygenase oder die Kombination aus einer rekombinanten Fettsäurereduktase und aus einer die Fettsäurereduktase phosphopantheteinylierenden Phosphopantheteinyl-Transferase exprimiert, und der zusätzlich eine Transaminase exprimiert, wobei die Phosphopantheteinyl-Transferase und/oder Transaminase bevorzugt rekombinant ist.

In einer ersten Ausführungsform des ersten Aspekts wird das Problem durch einen Ganzzellkatalysator gelöst, der zusätzlich eine Aminosäuredehydrogenase exprimiert, die bevorzugt rekombinant ist.

In einer zweiten Ausführungsform, die auch eine Ausführungsform der ersten Ausführungsform darstellt, wird das Problem durch einen Ganzzellkatalysator gelöst, der zusätzlich eine Alkanhydroxylase exprimiert, die bevorzugt rekombinant ist.

In einer dritten Ausführungsform, die auch eine Ausführungsform der ersten bis zweiten Ausführungsform darstellt, wird das Problem durch einen Ganzzellkatalysator gelöst, der zusätzlich ein Polypeptid der AlkL-Familie exprimiert, das bevorzugt rekombinant ist.

In einer vierten Ausführungsform, die auch eine Ausführungsform der zweiten Ausführungsform darstellt, wird das Problem durch einen Ganzzellkatalysator gelöst, der zusätzlich eine Alkoholdehydrogenase exprimiert, die bevorzugt rekombinant ist.

In einer fünften Ausführungsform, die auch eine Ausführungsform der ersten bis vierten Ausführungsform darstellt, wird das Problem durch einen Ganzzellkatalysator gelöst, wobei die Aktivität wenigstens eines an der β-Oxidation beteiligten Enzyms gegenüber dem Wildtyp des Ganzzellkatalysators verringert ist.

In einer sechsten Ausführungsform, die auch eine Ausführungsform der ersten bis fünften Ausführungsform darstellt, wird das Problem durch einen Ganzzellkatalysator gelöst, wobei die Aktivität von BioH oder einer Variante davon gegenüber dem Wildtyp des Ganzzellkatalysators verringert oder erhöht ist.

In einer siebten Ausführungsform, die auch eine Ausführungsform der ersten bis sechsten Ausführungsform darstellt, wird das Problem durch einen Ganzzellkatalysator gelöst, wobei die Aktivität von FadL oder einer Variante davon gegenüber dem Wildtyp des Ganzzellkatalysators erhöht ist.

In einem zweiten Aspekt wird das der Erfindung zu Grunde liegende Problem gelöst durch ein Verfahren zur Umsetzung einer Carbonsäure oder Dicarbonsäure oder eines Monoesters davon zu einem Amin oder Diamin, umfassend die Schritte
a) Bereitstellen einer Carbonsäure oder Dicarbonsäure oder eines Monoesters davon, für den Fall, dass es sich um eine Dicarbonsäure handelt, bevorzugt durch Kontaktieren einer Carbonsäure mit einer Alkanhydroxylase und/oder Alkoholdehydrogenase,
b) Kontaktieren des Carbonsäure oder Dicarbonsäure oder des Monoesters davon mit einer phosphopantheteinylierten Fettsäurereduktase oder einer α-Dioxygenase unter Bildung eines Aldehydproduktes, und
c) Kontaktieren des Produktes aus Schritt a) mit einer Transaminase.

In einem zweiten Aspekt wird das der Erfindung zu Grunde liegende Problem gelöst durch ein Verfahren, wobei bei Schritt c) eine Aminosäuredehydrogenase anwesend ist.

In einer ersten Ausführungsform des zweiten Aspekts wird das Problem gelöst durch ein Verfahren, wobei wenigstens ein Enzym aus der Gruppe umfassend phosphopantheteinylierte Fettsäurereduktase, α-Dioxygenase, Transaminase, Aminosäuredehydrogenase und Alkanhydroxylase, bevorzugt alle verwendeten Enzyme aus dieser Gruppe, in Form eines Ganzzellkatalysators dem ersten Aspekt der vorliegenden Erfindung bereitgestellt werden.

In einer zweiten Ausführungsform, die auch eine Ausführungsform der ersten Ausführungsform darstellt, wird das Problem gelöst durch ein Verfahren, wobei es sich bei der Carbonsäure oder Dicarbonsäure oder dem Monoester davon um eine Verbindung der Formel (I)

R¹ - A - COOR² (I)

handelt, wobei R¹ aus der Gruppe ausgewählt ist, die -H und COOR³ umfasst,
wobei R² und R³ jeweils und unabhängig voneinander aus der Gruppe ausgewählt sind, die H, Methyl, Ethyl und Propyl umfasst,
mit der Maßgabe, dass wenigstens einer der Reste R² und R³ H ist,
wobei A eine unverzweigte, verzweigte, lineare, zyklische, substituierte oder unsubstituierte Kohlenwasserstoffgruppe mit wenigstens vier Kohlenstoffatomen darstellt.

In einer dritten Ausführungsform, die auch eine Ausführungsform der ersten bis zweiten Ausführungsform darstellt, wird das Problem gelöst durch ein Verfahren, wobei A die Formel - (CH₂)ₙ - aufweist, wobei n wenigstens 4, bevorzugt wenigstens 10 ist.

In einem dritten Aspekt wird das der Erfindung zu Grunde liegende Problem gelöst durch eine Verwendung des Ganzzellkatalysators nach dem ersten Aspekt oder des Verfahrens nach dem zweiten Aspekt zur Aminierung einer Fettsäure, ω-Hydroxy-Fettsäure, ω-Oxo-Fettsäure oder einem Monoester davon.

In einem vierten Aspekt wird das der Erfindung zu Grunde liegende Problem gelöst durch eine Reaktionsmischung umfassend den Ganzzellkatalysator nach dem ersten Aspekt in wässriger Lösung sowie eine Fettsäure, ω-Hydroxy-Fettsäure, ω-Oxo-Fettsäure oder einen Monoester davon der Formel (I)

R¹ - A - COOR² (I),

wobei R¹ aus der Gruppe ausgewählt ist, die -H, -CHO, -OH und COOR³ umfasst,
wobei R² und R³ jeweils und unabhängig voneinander aus der Gruppe ausgewählt sind, die H, Methyl, Ethyl und Propyl umfasst,
mit der Maßgabe, dass wenigstens einer der Reste R² und R³ H ist,
wobei A eine unverzweigte, verzweigte, lineare, zyklische, substituierte oder unsubstituierte Kohlenwasserstoffgruppe mit wenigstens vier Kohlenstoffatomen darstellt, bevorzugt die Formel - (CH₂)ₙ -, wobei n wenigstens 4, besonders bevorzugt wenigstens 10 ist.

Die vorliegende Erfindung beruht auf der Erkenntnis der Erfinder, dass eine funktionell rekombinante Fettsäurereduktase oder α-Dioxygenase in einem Ganzzellkatalysator, der zur Herstellung von Aminen und Diaminen aus Fettsäuren verwendet wird und eine entsprechende Enzymausstattung aufweist, überraschend die Ausbeute an Aminen und Diaminen erhöht.

Weiterhin beruht die vorliegende Erfindung auf der Erkenntnis der Erfinder, dass eine funktionell rekombinante Fettsäurereduktase oder α-Dioxygenase in einem Ganzzellkatalysator, der zur Herstellung von Aminen und Diaminen aus Fettsäuren verwendet wird und eine entsprechende Enzymausstattung aufweist, überraschend die Konzentration störender Nebenprodukte, insbesondere von überoxidierten Fettsäuren in Form von Dicarbonsäuren und Estern davon, im anfallenden Produkt verringert.

Weiterhin beruht die vorliegende Erfindung auf der Erkenntnis der Erfinder, dass eine funktionell rekombinante Fettsäurereduktase oder α-Dioxygenase in einem Ganzzellkatalysator, der zur Herstellung von Aminen und Diaminen Fettsäuren verwendet wird und eine entsprechende Enzymausstattung aufweist, die Reinheit und Wiederverwendbarkeit von flüssigen Kationenaustauschern wie Ölsäure verbessert, die zur Entfernung des Amins und Diamins aus einem Fermentationslösung umfassend den Ganzzellkatalysator verwendet werden.

Die vorliegende Erfindung betrifft ein verbessertes Verfahren zur Umsetzung einer Carbonsäure oder Dicarbonsäure oder eines Monoesters davon zu einem Amin oder Diamin, das sich dadurch auszeichnet, dass neben den Enzymen, die die Überführung der Fettsäure über ihre verschiedenen Oxidationsstufen zum Amin katalysieren, auch eine Fettsäurereduktase oder α-Dioxygenase anwesend ist, bevorzugt, wenn ein Ganzzellkatalysator zur Durchführung des Verfahrens eingesetzt wird. In einer bevorzugten Ausführungsform wird unter dem Begriff "Fettsäurereduktase", wie hierin verwendet, ein Enzym verstanden, das die Umwandlung einer ω-Carboxysäure, auch als Dicarbonsäure oder ω-Carboxy-Fettsäure bezeichnet, zur entsprechenden ω-Oxofettsäure unter Verbrauch von ATP und NAD(P)H katalysiert. Im Stand der Technik, beispielsweise in der WO/2010/135624, sind Fettsäurereduktasen zur Herstellung von ω-Hydroxyfettsäuren beschrieben, nicht jedoch als Teil eines Systems zu Herstellung von ω-Aminofettsäuren. In einer noch bevorzugteren Ausführungsform ist die Fettsäurereduktase aus der Gruppe von Fettsäurereduktasen ausgewählt, die die Aminosäuresequenzen YP_887275.1, ZP_11001941.1, ZP_06852401.1, NP_959974.1, YP_001070587.1, ZP_05217435.1, YP_882653.1, YP_639435.1, ZP_10800193.1, YP_006452763.1, YP_006730440.1, ZP_11196216.1, YP_005349252.1, ZP_05224908.1, YP_005338837.1, YP_006307000.1, YP_005343991.1, ZP_11001942.1, ZP_09979565.1, YP_005003162.1, YP_953393.1, YP_001850422.1, ZP_11011489.1, ZP_12689264.1, YP_905678.1, ZP_09976919.1, YP_004746059.1, NP_217106.1, YP_004525443.1, NP_337166.1, ZP_09685823.1, YP_978699.1, ZP_06437984.1, ZP_06514086.1, NP_856267.1, CAA19077.1, NP_301424.1, ZP_06522140.1, ZP_06518098.1, ZP_11008938.1, ZP_07432374.2, AAR91681.1, YP_006808747.1, YP_001851230.1, ZP_15327751.1, ZP_15455857.1, ZP_12874284.1, ZP_15332534.1, ZP_15512956.1, ZP_14244106.1, ZP_15470899.1, ZP_11439367.1, YP_001703694.1, ZP_15446742.1, YP_006808978.1, ZP_07964926.1, YP_006521379.1, ZP_10796908.1, ZP_15512957.1, ZP_12874283.1, YP_005350955.1, ZP_14243341.1, YP_001705436.1, ZP_15329649.1, YP_006522325.1, YP_006732197.1, YP_003658971.1, ZP_05227804.1, YP_001703695.1, YP_006308707.1, ZP_15342047.1, YP_006521380.1, ZP_15327752.1, YP_005340557.1, ZP_11439578.1, ZP_15392943.1, ZP_15514789.1, ZP_12996178.1, ZP_09412214.1, ZP_06849686.1, YP_889972.1, YP_006570321.1, ZP_15375693.1, YP_006308219.1, YP_006521600.1, YP_005340029.1, YP_005350457.1, ZP_11439836.1, ZP_12994664.1, ZP_14240588.1, ZP_14236860.1, ZP_09410830.1, YP_006731697.1, YP_005264225.1, YP_001704097.1, ZP_15328186.1, ZP_09402885.1, ZP_12690463.1, AF059871.1, ZP_07966879.1, YP_118225.1, YP_001828302.1, YP_006566873.1, YP_003660169.1, ZP_15337407.1, ZP_08240521.1, ZP_10456477.1, YP_001537947.1, YP_004016539.1, ZP_07664024.1, ZP_14244107.1, ZP_09794557.1, ZP_09274211.1, ZP_05224899.1, ZP_15484175.1, AAA17105.1, ZP_11437924.1, ZP_15446621.1, YP_003646340.1, ZP_15382134.1, ZP_14237669.1, ZP_09165547.1, YP_004019203.1, ZP_14240225.1, YP_001220863.1, CBA74242.1, ZP_12994240.1, EIE27140.1, ZP_15354547.1, ZP_15432557.1, ZP_15500132.1, ZP_15478632.1, ZP_06846978.1, AAA17108.1, ZP_15333767.1, ZP_05217205.1, AAD44234.1, YP_005348984.1, YP_006306749.1, ZP_05224611.1, YP_005343772.1, YP_006730188.1, YP_882425.1, ZP_10799956.1, ZP_05045132.1, NP_960176.1, ZP_12398880.1, ZP_11192735.1, ZP_11440091.1, ZP_05217203.1, ZP_06846979.1, ZP_10800936.1, ZP_06523596.1, YP_882421.1, YP_006306748.1, YP_006522017.1, ZP_15432556.1, ZP_15354095.1, ZP_05227781.1, ZP_09684639.1, YP_006730187.1, YP_005343770.1, YP_005338616.1, YP_005348983.1, ZP_15472813.1, ZP_15457007.1, ZP_15421152.1, ZP_15488933.1, ZP_14240030.1, YP_001704825.1, ZP_15328982.1, YP_005911512.1, ZP_09411638.1, ZP_12876400.1, ZP_12995435.1, ZP_07667680.1, YP_001281387.1, EIE21044.1, ZP_15375054.1, Nu_334518.1, 4DQV_A, ZP_06435375.1, YP_003030020.1, YP_976237.1, ZP_04926822.1, YP_004998149.1, YP_004743589.1, YP_005907921.1, NP_214615.1, YP_001286047.1, ZP_06515541.1, ZP_05139482.1, YP_888016.1, ZP_06452908.1, ZP_06519578.1, YP_004721827.1, CAJ77696.1, ZP_09680854.1, ZP_09686453.1, YP_884815.1, YP_884815.1, CAB55600.1, ZP_09081423.1, YP_006521568.1, ZP_11440626.1, ZP_15513309.1, ZP_09410778.1, ZP_15374248.1, ZP_15405954.1, YP_001704047.1, ZP_14236911.1, ZP_12873916.1, ZP_14242094.1, ZP_12994610.1, ZP_07664023.1, ZP_15446620.1, ZP_15484174.1, ZP_14240245.1, YP_005358845.1 und XP_002669159.1, insbesondere YP_006731697.1, ZP_09839660.1, YP_001704097.1, YP_889972.1, ZP_05045132.1, ZP_09794557.1, ZP_08240521.1, NP_959974.1, ZP_10456477.1, YP_118225.1, NP_217106, YP_905678.1, YP_887275.1, ZP_11001941.1, YP_953393.1 und YP_005349252.1 Varianten davon umfassen.

Bei Fettsäurereduktasen handelt es sich um eine Gruppe von Enzymen, die für ihre Aktivität eine Phosphopantheteinylierung, d. h. die kovalente Befestigung eines Phosphopantheteinyl-Cofaktors am Enzym, benötigen. Dementsprechend ist die erfindungsgemäß verwendete Fettsäurereduktase phosphopantheteinyliert, und eine die Fettsäurereduktase exprimierender Ganzzellkatalysator exprimiert, entweder als Teil seiner Ausstattung endogen exprimierter Enzyme oder in rekombinanter Form, eine die Fettsäurereduktase phosphopantheteinylierende Phosphopantheteinyl-Transferase. In einer bevorzugten Ausführungsform wird unter dem Begriff "Phosphopantheteinyi-Transferase", wie hierin verwendet, ein Enzym verstanden, dass einen Phosphopantheteinyl-Rest von einem Phosphopantheteinyl-CoA auf ein Enzym, bevorzugt an der Fettsäurereduktase, überträgt. In einer besonders bevorzugten Ausführungsform ist die Phosphopantheteinyl-Transferase aus der Gruppe von Phosphopantheteinyl-Transferasen ausgewählt, die die Aminosäuresequenzen ABI83656.1, YP_006811024.1, YP_120266.1, YP_005265173.1, YP_004006671.1, ZP_08152482.1, ZP_11104141.1, ZP_14482198.1, YP_706581.1, ZP_10002626.1, ZP_09308410.1, YP_002783881.1, ZP_18276502.1, ZP_09271851.1, ZP_08204640.1, YP_002766085.1, ZP_09788717.1, ZP_09799863.1, ZP_10961877.1, YP_003273299.1, GAB86168.1, YP_006668875.1, ZP_08766535.1, ZP_09793386.1, ZP_09212827.1, ZP_09276344.1, ZP_09213870.1, ZP_09081490.1, ZP_10947586.1, YP_003658841.1, ZP_06852853.1, YP_953148.1, ZP_11011170.1, YP_639258.1, YP_886985.1, ZP_11194383.1, ZP_09681094.1, ZP_06455719.1, NP_337369.1, YP_004077819.1, NP_217310.1, YP_006452521.1, YP_005339056.1, ZP_05226335.1, ZP_07965127.1, ZP_07419314.2, NP_302077.1, YP_005003342.1, YP_005349465.1, ZP_10800435.1, ZP_06564430.1, YP_882860.1, YP_001135287.1, YP_001850220.1, ZP_05217634.1, YP_003646683.1, YP_004746246.1, ZP_15327906.1, ZP_09979035.1, YP_001703848.1, YP_906028.1, ZP_15395499.1, ZP_11438833.1, ZP_11005955.1, ZP_09410582.1, NP_961833.1, YP_001106197.1, ZP_14237113.1, YP_004085491.1, YP_003835595.1, ZP_12994399.1, YP_004523804.1, ZP_12690887.1, YP_003339468.1, ZP_06589331.1, YP_004801334.1, ZP_09974565.1, ZP_04608379.1, ZP_13037142.1, YP_712537.1, ZP_11236665.1, NP_630748.1, ZP_06527138.1, YP_003835167.1, CCH33620.1, ZP_10309401.1, ZP_08881396.1, YP_003102953.1, YP_003487252.1, ZP_08881565.1, YP_006263961.1, NP_822924.1, YP_004914569.1, ZP_09400366.1, AFV71333.1, ZP_07309518.1, ZP_09172171.1, ZP_06710898.1, CAN89630.1, ZP_06921116.1, ZP_08804003.1, ZP_19189663.1, ZP_10545589.1, YP_006248725.1, ZP_10455557.1, YP_004015869.1, ZP_08801530.1, ZP_10550999.1, YP_004492879.1, ZP_09958730.1, ZP_08286666.1, ZP_11212856.1, AAL15597.1, AAZ94407.1, ZP_19188802.1, AFF18625.1, ZP_06575404.1, AAK06801.1, ADC79635.1, YP_004080528.1, YP_004921314.1, ACY01405.1, YP_004584022.1, YP_003114157.1, YP_003203177.1, AFB69911.1, YP_006876460.1, ZP_08024798.1, YP_006269867.1, YP_006881814.1, CCK26150.1, ZP_07307765.1, ZP_07315112.1, YP_005466392.1, NP_824081.1, YP_003493882.1, ZP_06412387.1, ZP_10068239.1, ZP_08234258.1, YP_001822177.1, ZP_03979107.1, ZP_07979043.1, BAA22407.1, ZP_09402950.1, YP_003112617.1, NP_738483.1, YP_480609.1, EKX90208.1, BAE93744.1, BAB69186.1, ZP_04713061.1, YP_006881735.1, ZP_07274901.1, ZP_11379052.1, ZP_06581115.1, YP_006437406.1, ZP_12871839.1, NP_601186.1, ZP_08451808.1, YP_005057339.1, YP_005303909.1, ZP_07090824.1, YP_003783676.1, YP_004630011.1, ZP_06588772.1, AAX98203.1, AFK80329.1, ZP_08124665.1, ZP_03710365.1, AAB 17877.1, ZP_07403633.1, ZP_11268660.1, ZP_07288841.1, ABV83217.1, ZP_16178576.1, AAG43513.1, ZP_09155938.1, YP_004605750.1, ZP_03918977.1, AAF71762.1, ZP_05007864.1, ZP_06836265.1, ZP_03934882.1, YP_001508477.1, ZP_06043756.1, ZP_05366306.1, YP_002835056.1, ZP_03933464.1, ZP_07469321.1, ZP_07713507.1, YP_005160553.1, NP_939820.1, AAU93794.1, ZP_14659796.1, ZP_14383679.1, YP_005058606.1, YP_001221073.1, ZP_08231568.1, YP_250920.1, ZP_11383249.1, YP_003916320.1, ZP_08681170.1, YP_001800249.1, YP_001157632.1, YP_166099.1, ZP_10088015.1, YP_004760065.1, ZP_07947675.1, YP_001603066.1, YP_003812683.1, YP_004403402.1, ZP_08292153.1, ZP_09471260.1, YP_004018108.1, ZP_05115352.1, AAD13565.1, ZP_09295321.1, YP_001535629.1, ZP_04607273.1, YP_006561753.1, ZP_00960958.1, YP_006571985.1, ZP_08862188.1, YP_002906426.1, CCK30433.1, ZP_13042493.1, ZP_09090153.1, YP_614397.1, ZP_11163860.1, YP_003983492.1, YP_004080668.1, ZP_09420475.1, ZP_05914565.1, ZP_01101149.1, ZP 14743088.1, YP_001239694.1, ZP_09127532.1, YP_003833873.1, ZP_08516197.1, ZP_10160483.1, ZP_01987188.1, ZP_01755304.1, ZP_08825027.1, ZP_05077116.1, YP_001444606.1, ZP_03392800.1, ZP_01057781.1, AFB69889.1, ZP_08815097.1 und AAO17175.1 und Varianten davon umfassen. In einer besonders bevorzugten Ausführungsform handelt es sich dabei um die Phosphopantheteinyl-Transferase mit dem Datenbankcode ABI83656.1 oder eine Variante davon.

Alternativ oder zusätzlich zur Kombination aus Fettsäurereduktase und Phosphopantheteinyl-Transferase kann der Ganzzellkatalysator auch eine α-Dioxygenase aufweisen. In einer bevorzugten Ausführungsform wird unter dem Begriff "α-Dioxygenase", wie hierin verwendet, ein Enzym verstanden, dass die Umwandlung einer Carbonsäure und/oder Dicarbonsäure oder eines Monoesters davon unter Verbrauch eines Moleküls Sauerstoff und unter Abspaltung eines Kohlendioxid-Moleküls zu einer am endständigen ω-Kohlenstoffatom gegenüber der als Edukt eingesetzten Carbonsäure und/oder Dicarbonsäure oder eines Monoesters davon um ein Kohlenstoffatom verkürzten, am endständigen ω-Kohlenstoffatom eine Aldehydgruppe tragenden Carbonsäure und/oder Dicarbonsäure oder eines Monoesters davon katalysiert. In einer besonders bevorzugten Ausführungsform ist die α-Dioxygenase aus der Gruppe von α-Dioxygenasen ausgewählt, die die Aminosäuresequenzen NP_001066718.1, EAY82977.1, BAH79993.1, ABG22011.1, BAJ90503.1, AFD04418.1, AFD04417.1, BAJ87736.1, AFW75180.1, ABG22012.1, XP_002311389.1, CAH05011.1, XP_002279884.1, CBI34957.3, AAG59584.1, NP_001234414.1, NP_001234410.1, XP_003553942.1, XP_002275161.1, XP_003553937.1, CBI34960.3, CAA07589.1, XP_003543402.1, XP_002517402.1, XP_002882184.1, NP_186791.1, AAK85133.1, CAN77070.1, XP_002529555.1, CAH64542.1, NP_001234061.1, XP_002281357.1, ADM21465.1, XP_002318527.1, NP_177509.1, CAN74266.1, XP_002888940.1, NP_001185393.1, XP_003631072.1, BAJ33800.1, XP_002517377.1, XP_003530944.1, BAJ34623.1, ABG22013.1, ABP02610.1, XP_001773135.1, XP_002960339.1, ABK95279.1, ABD73303.1, ABD73304.1, YP_001805721.1, ZP_08971815.1, ZP_08430366.1, YP_823013.1, ZP_05026427.1, ZP_11003953.1, YP_007064484.1, YP_007113008.1, YP_633369.1, ZP_18906570.1, ZP_09251410.1, ZP_10050808.1, ZP_01306662.1, YP_001516886.1, ZP_05042862.1, AAC49625.1, ZP_09648375.1, ZP_09792714.1, ZP_09788527.1, XP_001728273.1, AAC83355.1, YP_890542.1, ZP_11000891.1, XP_002605323.1, EG058341.1, YP_006249145.1, YP_001507004.1, YP_001704637.1, ZP_12876141.1, ZP_11150830.1, ZP_14236257.1, ZP_09411385.1, ZP_14243118.1, EKD16664.1, ZP_15416799.1, ZP_15338016.1, ZP_10080295.1, ZP_11438929.1, ZP_12995210.1, ZP_10946648.1, YP_003409541.1, XP_001637870.1, YP_005451221.1, XP_001212758.1, ZP_07290489.1, ZP_05781329.1, ZP_19187748.1, ZP_06574534.1, XP_002605322.1, NP_822950.1, YP_006366425.1, EJP63377.1, EKD21217.1, XP_001795927.1, XP_003042615.1, ZP_06566152.1, EGU88116.1, EFY94417.1, XP_388327.1, EKJ68934.1, ZP_07290463.1, CCC10458.1, YP_001107201.1, XP_003348248.1, T49753, CAD31840.1, XP_001229975.1, CBN77040.1, YP_004813753.1, XP_002513273.1, XP_001627136.1, AFG52858.1, AFG52857.1, AEW08450.1, NP_841291.1, YP_004512343.1, ACG75701.1 und ZP_03500906.1 und Varianten davon umfassen. In einer besonders bevorzugten Ausführungsform handelt es sich um die α-Dioxygenase mit dem Datenbankcode NP_001066718.1 oder eine Variante davon.

Neben der α-Dioxygenase oder der Kombination aus Fettsäurereduktase und der Phosphopantheteinyl-Transferase weist der erfindungsgemäße Ganzzellkatalysator notwendigerweise eine Transaminase auf, die endständige Aldehydgruppen aminiert. In einer bevorzugten Ausführungsform wird unter dem Begriff "Transaminase", wie hierin verwendet, ein Enzym verstanden, das die Übertragung von α-Aminogruppen von einem Donor-, bevorzugt einer Aminosäure, auf ein Akzeptormolekül, bevorzugt eine α-Ketocarbonsäure, katalysiert. In einer besonders bevorzugten Ausführungsform ist die Transaminase aus der Gruppe von Transaminasen ausgewählt, die die Aminosäuresequenzen 3HMU_A, AAD41041.1, AAK15486.1, ABE03917.1, ADR60699.1, ADR61066.1, ADR62525.1, AEL07495.1, CAZ86955.1, EFW82310.1, EFW87681.1, EGC99983.1, EGD03176.1, EGE58369.1, EGH06681.1, EGH08331.1, EGH24301.1, EGH32343.1, EGH46412.1, EGH55033.1, EGH62152.1, EGH67339.1, EGH70821.1, EGH71404.1, EGH78772.1, EGH85312.1, EGH97105.1, EGP57596.1, NP_102850.1, NP_106560.1, NP_248912.1, NP_248990.1, NP_354026.2, NP_421926.1, NP_637699.1, NP_642792.1, NP_744329.1, NP_744732.1, NP_747283.1, NP_795039.1, NP_901695.1 (, XP_002943905.1, YP_001021095.1, YP_001059677.1, YP_001061726.1, YP_001066961.1, YP_001074671.1, YP_001120907.1, YP_001140117.1, YP_001170616.1, YP_001185848.1, YP_001188121.1, YP_001233688.1, YP_001268866.1, YP_001270391.1, YP_001345703.1, YP_001412573.1, YP_001417624.1, YP_001526058.1, YP_001579295.1, YP_001581170.1, YP_001668026.1, YP_001669478.1, YP_001671460.1, YP_001685569.1, YP_001747156.1, YP_001749732.1, YP_001765463.1, YP_001766294.1, YP_001790770.1, YP_001808775.1, YP_001809596.1, YP_001859758.1, YP_001888405.1, YP_001903233.1, YP_001977571.1, YP_002229759.1, YP_002231363.1, YP_002280472.1, YP_002297678.1, YP_002543874.1, YP_002549011.1, YP_002796201.1, YP_002801960.1, YP_002875335.1, YP_002897523.1, YP_002912290.1, YP_002974935.1, YP_003060891.1, YP_003264235.1, YP_003552364.1, YP_003578319.1, YP_003591946.1, YP_003607814.1, YP_003641922.1, YP_003674025.1, YP_003692877.1, YP_003755112.1, YP_003896973.1, YP_003907026.1, YP_003912421.1, YP_004086766.1, YP_004142571.1, YP_004147141.1, YP_004228105.1, YP_004278247.1, YP_004305252.1, YP_004356916.1, YP_004361407.1, YP_004378186.1, YP_004379856.1, YP_004390782.1, YP_004472442.1, YP_004590892.1, YP_004612414.1, YP_004676537.1, YP_004693233.1, YP_004701580.1, YP_004701637.1, YP_004704442.1, YP_108931.1, YP_110490.1, YP_168667.1, YP_237931.1, YP_260624.1, YP_262985.1, YP_271307.1, YP_276987.1, YP_334171.1, YP_337172.1, YP_350660.1, YP_351134.1, YP_364386.1, YP_366340.1, YP_369710.1, YP_370582.1, YP_426342.1 YP_440141.1, YP_442361.1, YP_468848.1, YP_521636.1, YP_554363.1, YP_608454.1, YP_610700.1, YP_614980.1, YP_622254.1, YP_625753.1, YP_680590.1, YP_751687.1, YP_767071.1, YP_774090.1, YP_774932.1, YP_788372.1, YP_858562.1, YP_928515.1, YP_983084.1, YP_995622.1, ZP_00948889.1, ZP_00954344.1, ZP_00959736.1, ZP_00998881.1, ZP_01011725.1, ZP_01037109.1, ZP_01058030.1, ZP_01076707.1, ZP_01103959.1, ZP_01167926.1, ZP_01224713.1, ZP_01442907.1, ZP_01446892.1, ZP_01550953.1, ZP_01625518.1, ZP_01745731.1, ZP_01750280.1, ZP_01754305.1, ZP_01763880.1, ZP_01769626.1, ZP_01865961.1, ZP_01881393.1, ZP_01901558.1, ZP_02145337.1, ZP_02151268.1, ZP_02152332.1, ZP_02167267.1, ZP_02190082.1, ZP_02242934.1, ZP_02360937.1, ZP_02367056.1, ZP_02385477.1, ZP_02456487.1, ZP_02883670.1, ZP_03263915.1, ZP_03263990.1, ZP_03400081.1, ZP_03452573.1, ZP_03456092.1, ZP_03517291.1, ZP_03529055.1, ZP_03571515.1, ZP_03572809.1, ZP_03587785.1, ZP_03588560.1, ZP_03697266.1, ZP_03697962.1, ZP_04521092.1, ZP_04590693.1, ZP_04890914.1, ZP_04891982.1, ZP_04893793.1, ZP_04902131.1, ZP_04905327.1, ZP_04941068.1, ZP_04944536.1, ZP_04945255.1, ZP_04959332.1, ZP_04964181.1, ZP_05053721.1, ZP_05063588.1, ZP_05073059.1, ZP_05077806.1, ZP_05082750.1, ZP_05091128.1, ZP_05095488.1, ZP_05101701.1, ZP_05116783.1, ZP_05121836.1, ZP_05127756.1, ZP_05637806.1, ZP_05742087.1, ZP_05783548.1, ZP_05786246.1, ZP_05843149.1, ZP_05945960.1, ZP_06459045.1, ZP_06487195.1, ZP_06492453.1, ZP_06493162.1, ZP_06703644.1, ZP_06731146.1, ZP_06839371.1, ZP_07007312.1, ZP_07266194.1, ZP_07374050.1, ZP_07662787.1, ZP_07778196.1, ZP_07797983.1, ZP_08099459.1, ZP_08138203.1, ZP_08141719.1, ZP_08142973.1, ZP_08177102.1, ZP_08185821.1, ZP_08186468.1, ZP_08208888.1, ZP_08266590.1, ZP_08402041.1, ZP_08406891.1, ZP_08522175.1, ZP_08527488.1, ZP_08631252.1, ZP_08636687 und Varianten davon umfassen.

Bei der erfindungsgemäß verwendeten Fettsäurereduktase und bevorzugt auch bei anderen erfindungsgemäß verwendeten Enzymen handelt es sich um rekombinante Enzyme. In einer bevorzugten Ausführungsform wird unter dem Begriff "rekombinant", wie hierin verwendet, verstanden, dass das für das entsprechende Enzym kodierende Nukleinsäuremolekül in der natürlichen Zelle nicht vorkommt und/oder es unter Verwendung von gentechnischen Methoden hergestellt wurde. In einer bevorzugten Ausführungsform spricht man von einem rekombinanten Protein, wenn das entsprechende Polypeptid von einer rekombinanten Nukleinsäure kodiert ist. In einer bevorzugten Ausführungsform wird unter einer rekombinanten Zelle, wie hierin verwendet, eine Zelle verstanden, die wenigstens eine rekombinante Nukleinsäure oder ein rekombinantes Polypeptid aufweist. Dem Fachmann sind zum Herstellen rekombinanter Moleküle oder Zellen geeignete Verfahren bekannt, beispielsweise die in Sambrook/Fritsch/Maniatis (1989): Molecular Cloning: A Laboratory Manual, Cold Spring Harbor Laboratory Press, 2nd edition, beschriebenen. Rekombinante Enzyme werden bevorzugt überexprimiert, beispielsweise unter Verwendung von pET- oder pGEX-Vektor-Systemen, die dem Fachmann bekannt sind.

Mit Hinblick auf die Wahl des Organismus unterliegt der erfindungsgemäß einsetzbare Ganzzellkatalysator keinen Einschränkungen, sofern er kultivierbar, stabil und ggf. gentechnisch einführbaren Modifikationen, z. B. Verfahren zur Abschwächung von Enzymaktivitäten, beispielsweise Knock outs, zugänglich ist. So kann es sich gleichermaßen um eine prokaryontische oder eukaryontische Zelle handeln. Im Falle einer eukaryontischen Zelle sind unizelluläre Eukaryonten besonders bevorzugt, besonders Hefen wie *Saccharomyces cerevisiae, Candida tropicalis, Candida albicans* und *Pichia pastoris.* Im Falle von prokaryontischen Zellen kann es sich beispielsweise um ein Bakterium handeln, das aus der Gruppe ausgewählt ist, die *Magnetococcus, Mariprofundus, Acetobacter, Acetobacterium, Acidiphilium, Afipia, Ahrensia, Asticcacaulis, Aurantimonas, Azorhizobium, Azospirillum, Bacillus, Bartonella, tribocorum, Beijerinckia, Bradyrhizobium, Brevundimonas, subvibrioides, Brucella, Caulobacter, Chelativorans, Citreicella, Citromicrobium, Clostridium, Corynebacterium, Dinoroseobacter, Erythrobacter, Fulvimarina, Gluconacetobacter, Granulibacter, Hirschia, Hoeflea, Hyphomicrobium, Hyphomonas, Ketogulonicigenium, Labrenzia, Loktanella, Magnetospirillum, Maricaulis, Maritimibacter, Mesorhizobium, Methylobacterium, Methylocystis, Methylosinus, Nitrobacter, Novosphingobium, Oceanibulbus, Oceanicaulis, Oceanicola, Ochrobactrum, Octadecabacter, Oligotropha, Paracoccus, Parvibaculum, Parvularcula, Pelagibaca, Phaeobacter, Phenylobacterium, Polymorphum, Pseudovibrio, Rhodobacter, Rhodomicrobium, Rhodopseudomonas, Rhodospirillum, Roseibium, Roseobacter, Roseomonas, Roseovarius, Ruegeria, Sagittula, Silicibacter, Sphingobium, Sphingomonas, Sphingopyxis, Starkeya, Sulfitobacter, Thalassiobium, Xanthobacter, Zymomonas, Agrobacterium, Rhizobium, Sinorhizobium, Anaplasma, Ehrlichia, Neorickettsia, Orientia, Rickettsia, Wolbachia, Bordetella, Burkholderia, Cupriavidus, Taiwanensis, Lautropia, Limnobacter, Polynucleobacter, Ralstonia, Chromobacterium, Eikenella, corrodens, Basfia, Kingella, Laribacter, Lutiella, Neisseria, Simonsiella, Achromobacter, Acidovorax, Alicycliphilus, Aromatoleum, Azoarcus, Comamonas, Dechloromonas, Delftia, Gallionella, Herbaspirillum, Herminiimonas, Hylemonella, Janthinobacterium, Leptothrix, Methylibium, Methylobacillus, Methylophilales, Methyloversatilis, Methylovorus, Nitrosomonas, Nitrosospira, Oxalobacter, Parasutterella, Polaromonas, Polaromonas, Pusillimonas, Rhodoferax, Rubrivivax, Sideroxydans, Sutterella, wadsworthensis, Taylorella, Thauera, Thiobacillus, Thiomonas, Variovorax, Verminephrobacter, Anaeromyxobacter, Bdellovibrio, bacteriovorus, Bilophila, Desulfarculus, Desulfatibacillum, Desulfobacca, Desulfobacterium, Desulfobulbus, Desulfococcus, Desulfohalobium, Desulfitobacterium, Desulfomicrobium, Desulfonatronospira, Desulfotalea, Desulfovibrio, Desulfuromonas, Geobacter, Haliangium, Hippea, Lawsonia, Myxococcus, Pelobacter, Plesiocystis, Sorangium, Stigmatella, Syntrophobacter, Syntrophus, Arcobacter, Caminibacter, Campylobacter, Helicobacter, Nitratifractor, Nitratiruptor, Sulfuricurvum, Sulfurimonas, Sulfurospirillum, Sulfurovum, Wolinella, Buchnera, Blochmannia, Hamiltonella, Regiella, Riesia, Citrobacter, Cronobacter, Dickeya, Edwardsiella, Enterobacter, Erwinia, Escherichia, Klebsiella, Pantoea, Pectobacterium, Proteus, Providencia, Rahnella, Salmonella, Serratia, Shigella, Sodalis, Wigglesworthia, Glossina, Xenorhabdus, Yersinia, Acidithiobacillus, Acinetobacter, Aeromonas, Alcanivorax, Alkalilimnicola, Allochromatium, Alteromonadales, Alteromonas, Baumannia, Beggiatoa, Bermanella, Carsonella, Ruthia, Vesicomyosocius, Cardiobacterium, Chromohalobacter, Colwellia, Congregibacter, Coxiella, Dichelobacter, Endoriftia, Enhydrobacter, Ferrimonas, Francisella, Glaciecola, Hahella, Halomonas, Halorhodospira, Halothiobacillus, Idiomarina, Kangiella, Legionella, Marinobacter, Marinomonas, Methylobacter, Methylococcus, Methylomicrobium, Methylophaga, Moraxella, Moritella, Neptuniibacter, Nitrococcus, Pseudoalteromonas, Psychrobacter, Psychromonas, Reinekea, Rickettsiella, Saccharophagus, Shewanella, Succinatimonas, Teredinibacter, Thioalkalimicrobium, Thioalkalivibrio, Thiomicrospira, Tolumonas, Vibrionales, Actinobacillus, Aggregatibacter, Gallibacterium, Haemophilus, Histophilus, Mannheimia, Pasteurella, Azotobacter, Cellvibrio, Pseudomonas, Aliivibrio, Grimontia, Photobacterium, Photobacterium, Vibrio, Pseudoxanthomonas, Stenotrophomonas, Xanthomonas, Xylella, Borrelia, Brachyspira, Leptospira, Spirochaeta, Treponema, Hodgkinia, Puniceispirillum, Liberibacter, Pelagibacter, Odyssella, Accumulibacter,* insbesondere *B. subtilis, B. megaterium, C. glutamicum, E. coli, Pseudomonas sp., Pseudomonas fluorescens, Pseudomonas putida, Pseudomonas stutzeri" Acinetobacter* sp., *Burkholderia* sp., *Burkholderia thailandensis,* Cyanobakterien, *Klebsiella* sp., *Klebsiella oxytoca, Salmonella sp., Rhizobium sp.* und *Rhizobium meliloti,* umfasst. In einer besonders bevorzugten Ausführungsform handelt es sich um ein Enterobakterium, am bevorzugtesten um *Escherichia coli.*

Es ist vorteilhaft, wenn der erfindungsgemäße Ganzzellkatalysator neben der Fettsäurereduktase, Phosphopantheteinyl-Transferase und der Transaminase auch eine Alanindehydrogenase aufweist, um das von der Transaminase bei der Aminierung der endständigen Aldehydgruppe verbrauchte Alanin aus anorganischen stickstoffhaltigen Molekülen zu regenerieren. In einer bevorzugten Ausführungsform wird unter dem Begriff "Alanindehydrogenase", wie hierein verwendet, ein Enzym verstanden, das die Umwandlung von L-Alanin unter Verbrauch von Wasser und NAD⁺ zu Pyruvat, Ammoniak und NADH und die umgekehrte Reaktion katalysiert. In einer besonders bevorzugten Ausführungsform ist die Alanindehydrogenase aus der Gruppe von Alanindehydrogenasen ausgewählt, die die Aminosäuresequenz der Alanindehydrogenase aus *Bacillus subtilis* (Datenbankcode L20916), *Rhizobium leguminosarum* (Datenbankcode CP001622), *Vibrio proteolyticus* (Datenbankcode AF070716), *Mycobacterium tuberculosis* (Datenbankcode X63069), *Enterobacter aerogenes* (Datenbankcode AB013821), EGR93259.1, YP_003654745.1, YP_003651439.1, YP_003637111.1, YP_003631815.1, YP_001327051.1, YP_001262560.1, YP_886996.1, YP_882850.1, YP_704410.1, YP_703508.1, ZP_08624689.1, YP_001230376.1, P17557.1, P17556.1, CCB94892.1, CCB73698.1, YP_001168635.1, YP_004668736.1, YP_004569425.1, YP_003513168.1, YP_004561169.1, ZP_08554945.1, YP_400777.1, ZP_08311476.1, ZP_08310170.1, ZP_08267322.1, ZP_08263846.1, ZP_07898723.1, YP_149301.1, YP_148605.1, YP_004340432.1, EFT09946.1, EFS80513.1, EFS51332.1, EFS42459.1, YP_003060895.1, YP_003059033.1, ZP_03305373.1, YP_847214.1, YP_004095847.1, YP_003338282.1, YP_003337256.1, YP_355846.1, YP_253131.1, ZP_08197563.1, ZP_08196283.1, ADW06447.1, YP_734091.1, NP_372233.1, NP_102173.1, ZP_08170259.1, EGD36706.1, EGD32748.1, ZP_08155540.1, YP_004142849.1, YP_002417649.1, YP_001301040.1, YP_002992892.1, YP_081348.1, YP_080482.1, YP_002476349.1, ZP_08115025.1, ZP_08114403.1, YP_003552869.1, YP_002358112.1, YP_575010.1, YP_477594.1, YP_474564.1, YP_130399.1, YP_129373.1, YP_123314.1, NP_810467.1, NP_646469.1, NP_626044.1, NP_391071.1 (kodiert durch SEQ ID NR: 11), ZP_08086822.1, ZP_08084776.1, ZP_08083119.1, ZP_08020768.1, ZP_08013590.1, ZP_08011832.1, YP_003783744.1, YP_002781576.1, YP_002780533.1, ZP_02195873.1, NP_797482.1, ZP_07645051.1, ZP_07643260.1, ZP_06611917.1, AAT40119.1, ZP_07864946.1, YP_004068409.1, YP_002796203.1, YP_002774420.1, YP_003600348.1, YP_003599946.1, YP_003565624.1, YP_003565223.1, YP_335198.1, YP_423850.1, YP_155059.1, ZP_07843538.1, ZP_07841226.1, ZP_06928932.1, ZP_05692073.1, ZP_05687006.1, ZP_04867480.1, YP_775531.1, CBE70214.1, ZP_07721182.1, ZP_04302850.1, ZP_04298961.1, ZP_04287684.1, ZP_04277177.1, ZP_04248389.1, ZP_04235899.1, ZP_02159718.1, ZP_02152178.1, YP_003974610.1, YP_003546595.1, YP_002317127.1, ZP_07313778.1, ZP_07302778.1, ZP_07298850.1, CBK69442.1, YP_003413835.1, YP_003595089.1, ZP_06807811.1, YP_003582455.1, YP_003464731.1, YP_003496397.1, YP_003421918.1, CBL07274.1, CBK64956.1, YP_003508515.1, AAL87460.1, AAC23579.1, AAC23578.1, AAC23577.1, ACU78652.1, YP_003471439.1, YP_003452777.1, ZP_06384971.1, ACY25368.1, ABC26869.1, AAP44334.1, EEZ80018.1, ZP_05110458.1, 1PJB_A, ZP_04717201.1, ZP_04689103.1, CAO90307.1 CAM75354.1, CAA44791.1, BAA77513.1, EGR96638.1, EGL90046.1, YP_004510847.1, ZP_08450330.1, YP_003387804.1, YP_003058152.1, EFS74272.1, EFS67128.1, ZP_06844564.1, YP_826658.1, YP_001195249.1, YP_003095978.1, YP_469292.1, YP_004442054.1, YP_004461174.1, YP_004055616.1, YP_003576656.1, YP_003094537.1, YP_001295973.1, AEE71143.1, YP_004447480.1, YP_003761844.1, YP_040853.1, YP_003154888.1, YP_003142045.1, YP_002280953.1, NP_371963.1, NP_422368.1, EGC98966.1, EGC76398.1, YP_004263661.1, YP_004252039.1, YP_679036.1, YP_499973.1, ZP_08054972.1, ZP_08053009.1, ZP_04067276.1, ZP_03968868.1, ZP_03963857.1, ZP_03933079.1, ZP_03497046.1, ZP_06668924.1, ZP_06667106.1, ZP_06324464.1, ZP_06196777.1, ZP_05114159.1, ZP_05083968.1, ZP_05070370.1, ZP_05030022.1, ZP_04673064.1, ZP_03517011.1, ZP_03505783.1, XP_001310698.1, ABK27691.1 und CAB59281.2 und Varianten davon umfassen. Für die von der Alanindehydrogenase katalysierte Reaktion ist die Anwesenheit nicht nur von Pyruvat, das als Teil des Primärstoffwechsels von jeder als Ganzzellkatalysator in Frage kommenden Zelle gebildet wird, erforderlich, sondern auch von Ammonium. Letzteres wird typischerweise in Form von anorganischen Stickstoffsalzen, beispielsweise Ammoniumsalzen, Nitraten o. ä. bereitgestellt. Bevorzugt wird dem wässrigen Reaktionsmedium ein Ammoniumsalz zugesetzt, z.B. Ammoniumchlorid.

Weiterhin ist es vorteilhaft, wenn der erfindungsgemäße Ganzzellkatalysator eine Alkanhydroxylase und optional weitere, für die Aktivität der Alkanhydroxylase essentielle Enzyme exprimiert, insbesondere für den Fall, dass als Substrate zur Herstellung eines Diamins eine Fettsäure mit nur einer endständigen Carboxyfunktion eingesetzt wird. Die Alkanhydroxylase und/oder eine zusätzlich exprimierte Alkoholdehydrogenase oxidieren das endständige Kohlenstoffatom dann bis zur Aldehydgruppe, die anschließend von der Transaminase aminiert werden kann, oder bis zur Carboxygruppe, die durch eine α-Dioxygenase oder die Kombination aus Fettsäurereduktase und einer Phosphopantheteinyl-Transferase in eine endständige Aldehydgruppe umgewandelt wird, welche anschließend von der Transaminase aminiert werden kann. In einer bevorzugten Ausführungsform wird unter dem Begriff "Alkanhydroxylase", wie hierin verwendet, ein Enzym verstanden, das die Hydroxylierung von unsubstituierten linearen Alkylresten umfassend wenigstens sechs, bevorzugt zwölf Kohlenstoffstoffreste katalysiert.

Als Alkanhydroxylasen sind erfindungsgemäß zahlreiche Oxidationssysteme geeignet, wie sie u. a. in der PCT/EP 2008/067447 beschrieben sind. In einer bevorzugten Ausführungsform handelt es sich bei der Alkanhydroxylase um eine Cytochrom P450-Monooxygenase der CYP153-Familie. In einer bevorzugten Ausführungsform wird unter dem Begriff "Cytochrom P450-Monooxygenase der CYP153-Familie" eine cytosolische Oxidase verstanden, die Teil eines 3 Komponenten-Systems ist, das weiterhin ein Ferredoxin und eine Ferredoxin-Reduktase umfasst, mit einer Alkan-Bindestelle und der Fähigkeit, Alkane zu hydroxylieren. In einer besonders bevorzugten Ausführungsform handelt es sich um ein Enzym, das zu wenigstens 80, bevorzugt 90, am bevorzugtesten 95 oder 99 Prozent Sequenzidentität zur Cytochrom P450-Monooxygenase der CYP153-Familie aus *Alcanivorax borkumensis* SK2 (Datenbankcode YP_691921) aufweist oder um ein Enzym, das eine Polypeptidsequenz umfasst, die wenigstens 80, bevorzugt 90, am bevorzugtesten 95 oder 99 Prozent Sequenzidentität zur Cytochrom P450-Monooxygenase der CYP153-Familie aus *Alcanivorax borkumensis* SK2 (Datenbankcode YP_691921) aufweist und darüber hinaus Alkanhydroxylase-Aktivität aufweist. Die genannten Datenbankcodes beziehen sich dabei, wie durchgehend in dieser Anmeldung, auf die NCBI (National Center for Biotechnology Information, Bethesda, USA)-Datenbanken, genauer gesagt die am 21. November 2012 online verfügbare Version. In einer bevorzugten Ausführungsform wird unter dem Begriff "Cytochrom P450-Monooxygenase der CYP153-Familie" eine nicht membrangebundene Oxidase verstanden, die eine Bindestelle für Alkane, unsubstituierte lineare Alkylreste umfassend wenigstens fünf, bevorzugt zwölf Kohlenstoffstoffreste oder einfach hydroxylierte Alkane und deren Polypeptidkette das Motiv LL(I/L)(V/I)GGNDTTRN umfasst. In einer bevorzugten Ausführungsform handelt es sich bei einer "Cytochrom P450-Monooxygenase der CYP153-Familie", wie hierin verwendet, um eine Cytochrom P450-Monooxygenase der CYP153-Familie aus *Alcanivorax borkumensis* SK2 (Datenbankcode YP_691921) oder eine Variante davon, die bevorzugt Alkanhydroxylaseaktivität aufweist.

Zur optimalen Versorgung der Cytochrom P450-Monooxygenase der CYP153-Familie mit Elektronen aus dem Reduktionsmittel, bevorzugt NADH, ist es bevorzugt, dass die Zelle die Alkanhydroxylase zusammen mit funktionell mit ihr wechselwirkender Ferredoxin-Reduktase und funktionell mit ihr wechselwirkendem Ferredoxin exprimiert wird. Dabei kann es sich um isolierte oder bei der Verwendung eines Ganzzellkatalysators um co-exprimierte Polypeptide oder um N- oder C-terminal mit der Cytochrom P450-Monooxygenase der CYP153-Familie fusionierte Polypeptide handeln. Ob eine Ferredoxin-Reduktase oder ein Ferredoxin mit einer gegebenen Cytochrom P450-Monooxygenase der CYP153-Familie mit einander funktionell wechselwirken, kann der Fachmann leicht dadurch feststellen, ob das Reduktionsmittel in Gegenwart eines Alkansubstrates und der drei Polypeptide effizienter als für den Fall, dass wenigstens eines der drei fehlt, oxidiert wird. Alternativ kann der von Scheps, D., Malca, H., Hoffmann, B., Nestl, B. M, und Hauer, B. (2011) Org. Biomol. Chem., 9, 6727 beschriebene Enzymtest verwendet werden, der im Fall funktionell wechselwirkender Polypeptide eine deutliche Erhöhung der Reaktionsgeschwindigkeit zeigt. In einer besonders bevorzugten Ausführungsform stammen die Cytochrom P450-Monooxygenase der CYP153-Familie, das Ferredoxin und die Ferredoxin-Reduktase aus dem gleichen Organismus. In einer besonders bevorzugten Ausführungsform handelt es sich um die Ferredoxin-Reduktase aus *Alcanivorax borkumensis* SK2 (Datenbankcode YP_691923) oder eine Variante davon, das Ferredoxin aus *Alcanivorax borkumensis* SK2 (Datenbankcode YP_691920) oder eine Variante davon und die Cytochrom P450-Monooxygenase der CYP153-Familie aus *Alcanivorax borkumensis* SK2 (Datenbankcode AP_691921) oder eine Variante davon.

In einer weiteren bevorzugten Ausführungsform handelt es sich bei der Alkanhydroxylase um eine AlkB-Monooxygenase. AlkB stellt eine zunächst aus dem AlkBGT-System aus *Pseudomonas putida* Gpo1 bekannt gewordene Oxidoreduktase dar, die von zwei weiteren Polypeptiden, AlkG und AlkT, abhängig ist. AlkT wird als FAD-abhängige Rubredoxin-Reduktase charakterisiert, die Elektronen aus NADH an AlkG weitergibt. Bei AlkG handelt es sich um ein Rubredoxin, ein eisenhaltiges Redoxprotein, das als direkter Elektronendonor für AlkB fungiert. In einer bevorzugten Ausführungsform wird unter dem Begriff "AlkB-Monooxygenase" ein Polypeptid mit einer Sequenzhomologie von wenigstens, in der Reihenfolge zunehmender Bevorzugung angegeben, 75, 80, 85, 90, 92, 94, 96, 98 oder 99 % zur Sequenz des AlkB von *Pseudomonas putida* Gpo1 (Datenbankcode: CAB54050.1; dieser Datenbankcode stammt wie alle weiteren in der Anmeldung verwendeten aus dem Stand der Technik, nämlich aus der NCBI Datenbank, genauer dem am 15. Oktober 2012 online verfügbaren Release) mit der Fähigkeit, Alkane zu oxidieren. In einer besonders bevorzugten Ausführungsform handelt es sich bei der AlkB-Monooxygenase um eine mit den AlkG (CAB54052.1)- und AlkT (CAB54063.1)-Polypeptiden aus *Pseudomonas putida* Gpo1 funktionell zusammenwirkende, Alkane oxidierende Oxidoreduktase. Zur optimalen Versorgung der AlkB-Alkanhydroxylase mit Elektronen ist es bevorzugt, dass die Zelle die Alkanhydroxylase zusammen mit funktionell mit ihr wechselwirkenden Hilfsproteinen, bevorzugt AlkG und/oder AlkT oder jeweils Varianten davon exprimiert wird, wobei es sich in einer besonders bevorzugten Ausführungsform wiederum um AlkG (CAB54052.1)- und AlkT (CAB54063.1)-Polypeptide aus *Pseudomonas putida* Gpo1 handelt.

Bei der Verwendung eines Ganzzellkatalysators kann sich das Problem stellen, dass ein Substrat mit einem intrazellulär lokalisierten Enzym in Kontakt gebracht werden muss, damit es zur erwünschten Reaktion kommt. Im Falle langkettiger Alkane und Derivate davon ist bevorzugt, dass der Ganzzellkatalysator ein Polypeptid der AlkL-Familie aufweist. AlkL ist ein Membranprotein aus *Pseudomonas putida,* das langkettige Fettsäuren und Derivate davon in bakterielle Zellen importieren kann. In einer bevorzugten Ausführungsform handelt es sich bei einem "Polypeptid der AlkL-Familie", wie hierin verwendet, um ein Polypeptid, das über eine Länge von 230 aufeinander abfolgenden Aminosäuren eine wenigstens 80, bevorzugt 90, noch bevorzugter 90%ige Sequenzidentität zu AlkL aus *Pseudomonas putida* (Datenbankcode CAB69081) oder eine Variante von AlkL aus *Pseudomonas putida* und bevorzugt die Fähigkeit aufweist, den Import von langkettigen Alkanen ins Innere einer Zelle zu unterstützen. In einer weiteren Ausführungsform handelt es sich bei einem "Polypeptid der AlkL-Familie", wie hierin verwendet, um eine in der äußeren Membran eines Gram-negativen Bakteriums lokalisiertes Polypeptid, welches das Sequenzmotiv DXWAPAXQ(V/A)GXR, aufweist, wobei X eine proteinogene Aminosäure darstellt, und bevorzugt zusätzlich AlkL aus *Pseudomonas putida* (Datenbankcode CAB69081) oder eine Variante davon ist. Beispielhafte Mitglieder der AlkL-Familie umfassen AlkL aus *Pseudomonas putida* (Datenbankcode CAB69081), *Marinobacter aquaeolei* VT8 (Datenbankcode YP_957722), *Oceanicaulis alexandrii* HTCC2633 (Datenbankcode ZP_00953584), *Marinobacter manganoxydans* Mn17-9 (Datenbankcode ZP_09158756), *Caulobacter* sp. K31 (Datenbankcode YP_001672217), *Pseudomonas oleovorans* (Datenbankcode Q00595) und Varianten davon.

Die Lehre der vorliegenden Erfindung kann nicht nur unter Verwendung von Makromolekülen mit der exakten Aminosäure- oder Nukleinsäuresequenz, auf die hierin Bezug genommen wird, bzw. nicht nur unter Verwendung von einer Zelle mit relativ zum jeweiligen Wildtyp verringerter Aktivität eines Polypeptides mit der exakten Aminosäuresequenz, auf die hierin Bezug genommen wird, ausgeführt werden, sondern auch unter Verwendung einer Variante derartiger Makromoleküle oder von einer Zelle mit einer relativ zum jeweiligen Wildtyp der jeweiligen Zelle verringerten Aktivität einer Variante des Polypeptids, die durch Deletion, Addition oder Substitution einer oder mehr als einer Aminosäuren oder Nukleinsäuren erhalten werden kann. In einer bevorzugten Ausführungsform bedeutet der Begriff "Variante" einer Nukleinsäuresequenz oder Aminosäuresequenz, im Folgenden gleichbedeutend und austauschbar mit dem Begriff "Homologon" gebraucht, wie hierin verwendet, eine andere Nukleinsäure- oder Aminosäuresequenz, die eine Sequenz umfasst oder darstellt, welche mit Hinblick auf die entsprechende ursprüngliche Wildtyp-Nukleinsäure- oder -aminosäuresequenz eine Homologie, hier gleichbedeutend mit Identität verwendet, von 70, 75, 80, 85, 90, 92, 94, 96, 98, 99 % oder mehr Prozent aufweist, wobei bevorzugt andere als die das katalytisch aktive Zentrum ausbildende Aminosäuren oder für die Struktur oder Faltung essentielle Aminosäuren deletiert oder substituiert sind oder solche lediglich konservativ substituiert sind, beispielsweise ein Glutamat statt einem Aspartat oder ein Leucin statt einem Valin. Der Stand der Technik beschreibt Algorithmen, die verwendet werden können, um das Ausmaß von Homologie von zwei Sequenzen zu berechnen, z. B. Arthur Lesk (2008), Introduction to bioinformatics, 3^{rd} edition. In einer weiteren bevorzugteren Ausführungsform der vorliegenden Erfindung weist die Variante einer Aminosäure- oder Nukleinsäuresequenz, bevorzugt zusätzlich zur oben genannten Sequenzhomologie, im Wesentlichen die gleiche enzymatische Aktivität des Wildtypmoleküls bzw. des ursprünglichen Moleküls auf. Zum Beispiel weist eine Variante eines als Protease enzymatisch aktiven Polypeptids die gleiche oder im Wesentlichen die gleiche proteolytische Aktivität wie das Polypeptidenzym auf, d.h. die Fähigkeit, die Hydrolyse einer Peptidbindung zu katalysieren. In einer besonderen Ausführungsform bedeutet der Begriff "im Wesentlichen die gleiche enzymatische Aktivität" eine Aktivität mit Hinblick auf die Substrate des Wildtyp-Polypeptids, die deutlich über der Hintergrundaktivität liegt oder/und sich um weniger als 3, bevorzugter 2, noch bevorzugter eine Größenordnung von den K_{M}- und/oder k_{cat}-Werten unterscheidet, die das Wildtyppolypeptid mit Hinblick auf die gleichen Substrate aufweist. In einer weiteren bevorzugten Ausführungsform umfasst der Begriff "Variante" einer Nukleinsäure- oder Aminosäuresequenz wenigstens einen aktiven Teil/oder Fragment der Nukleinsäure- bzw. Aminosäuresequenz. In einer weiteren bevorzugten Ausführungsform bedeutet der Begriff "aktiver Teil", wie hierin verwendet, eine Aminosäuresequenz oder eine Nukleinsäuresequenz, die eine geringere als die volle Länge der Aminosäuresequenz aufweist bzw. für eine geringere als die volle Länge der Aminosäuresequenz kodiert, wobei die Aminosäuresequenz oder die kodierte Aminosäuresequenz mit geringerer Länge als der Wildtyp-Aminosäuresequenz im Wesentlichen die gleiche enzymatische Aktivität wie das Wildtyppolypeptid oder eine Variante davon aufweist, beispielsweise als Protease. In einer besonderen Ausführungsform umfasst der Begriff "Variante" einer Nukleinsäure eine Nukleinsäure, deren komplementärer Strang, bevorzugt unter stringenten Bedingungen, an die Wildtyp-Nukleinsäure bindet. Die Stringenz der Hybridisierungsreaktion ist für den Fachmann leicht bestimmbar und hängt im Allgemeinen von der Länge der Sonde, den Temperaturen beim Waschen und der Salzkonzentration ab. Im Allgemeinen benötigen längere Sonden höhere Temperaturen zum Hybridisieren, wohingegen kürzere Proben mit geringen Temperaturen auskommen. Ob Hybridisierung stattfindet, hängt im Allgemeinen von der Fähigkeit der denaturierten DNA ab, an komplementäre Stränge zu annellieren, die in ihrer Umgebung vorhanden sind, und zwar unterhalb der Schmelztemperatur. Die Stringenz von Hybridisierungsreaktion und entsprechende Bedingungen sind ausführlicher in F M Ausubel (1995), Current Protocols in Molecular Biology. John Wiley & Sons, Inc. beschrieben. Anleitungen zur Identifizierung von DNA-Sequenzen mittels Hybridisierung findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology 41: 255-260 (1991)). Die Hybridisierung findet in einer bevorzugten Ausführungsform unter stringenten Bedingungen statt, das heißt, es werden nur Hybride gebildet, bei denen Sonde und Zielsequenz, d. h. die mit der Sonde behandelten Polynukleotide, mindestens 70% identisch sind. Es ist bekannt, dass die Stringenz der Hybridisierung einschließlich der Waschschritte durch Variieren der Pufferzusammensetzung, der Temperatur und der Salzkonzentration beeinflusst bzw. bestimmt wird. Die Hybridisierungsreaktion wird im Allgemeinen bei relativ niedriger Stringenz im Vergleich zu den Waschschritten durchgeführt (Hybaid Hybridisation Guide, Hybaid Limited, Teddington, UK, 1996). Für die Hybridisierungsreaktion kann beispielsweise ein Puffer entsprechend 5x SSC-Puffer bei einer Temperatur von ca. 50°C - 68°C eingesetzt werden. Dabei können Sonden auch mit Polynukleotiden hybridisieren, die weniger als 70% Identität zur Sequenz der Sonde aufweisen. Solche Hybride sind weniger stabil und werden durch Waschen unter stringenten Bedingungen entfernt. Dies kann beispielsweise durch Senken der Salzkonzentration auf 2x SSC und gegebenenfalls nachfolgend 0,5x SSC (The DIG System User's Guide for Filter Hybridisation, Boehringer Mannheim, Mannheim, Deutschland, 1995) erreicht werden, wobei eine Temperatur von in der Reihenfolge zunehmender Bevorzugung ca. 50°C - 68°C, ca. 52°C - 68°C, ca. 54°C - 68°C, ca. 56°C - 68°C, ca. 58°C - 68°C, ca. 60°C - 68°C, ca. 62°C - 68°C, ca. 64°C - 68°C, ca. 66°C - 68°C eingestellt wird. Temperaturbereiche von ca. 64°C - 68°C oder ca. 66°C - 68°C werden bevorzugt. Es ist gegebenenfalls möglich die Salzkonzentration bis auf eine Konzentration entsprechend 0,2 x SSC oder 0,1 x SSC zu senken. Durch schrittweise Erhöhung der Hybridisierungstemperatur in Schritten von ca. 1 - 2°C von 50°C auf 68°C können Polynukleotidfragmente isoliert werden, die beispielsweise in der Reihenfolge zunehmender Bevorzugung mindestens 70% oder mindestens 80% oder mindestens 90%, mindestens 91%, mindestens 92%, mindestens 93%, mindestens 94%, mindestens 95%, mindestens 96%, mindestens 97%, mindestens 98% oder mindestens 99% Identität zur Sequenz des eingesetzten Nukleinsäuremoleküls. Weitere Anleitungen zur Hybridisierung sind in Form sogenannter Kits am Markt erhältlich (z.B. DIG Easy Hyb von der Firma Roche Diagnostics GmbH, Mannheim, Deutschland, Catalog No. 1603558). In einer bevorzugten Ausführungsform umfasst der Begriff "Variante" einer Nukleinsäure, wie hierin verwendet, eine beliebige Nukleinsäuresequenz, die für die gleiche Aminosäuresequenz wie die ursprüngliche Nukleinsäure oder eine Variante dieser Aminosäuresequenz im Rahmen der Degeneriertheit des genetischen Codes kodiert.

In einer bevorzugten Ausführungsform weist die erfindungsgemäß verwendete Zelle eine ihrem Wildtyp gegenüber verringerte Aktivität wenigstens eines Enzyms aufweist, das eine der Reaktionen der β-Oxidation von Fettsäuren katalysiert, wobei es sich bevorzugt um ein Enzym aus der Gruppe handelt, die Fettsäure-CoA-Ligase, Acyl-CoA-Dehydrogenase, 2,4-Dienoyl-CoA-Reduktase, Enoyl-CoA-Hydratase and 3-Ketoacyl-CoA-Thiolase, einen Fettsäureimporter oder Varianten davon umfasst. Die β-Oxidation von Fettsäuren ist ein weit verbreiteter Stoffwechselweg, der es prokaryontischen und eukaryontischen Organismen gleichermaßen erlaubt, Fettsäuren zu oxidieren und die darin enthaltene chemische Energie dem Stoffwechsel verfügbar zu machen. Im weiteren Sinn beginnt sie mit der Aufnahme einer Fettsäure in die Zelle. Dort wird die Fettsäure, sofern die Bedingungen es erfordern, zunächst an der β-Position des CoA-Fettsäureesters durch eine Acyl-CoA-Dehydrogenase, im Falle von *E. coli* FadE, oxidiert. Ein ähnliches Molekül kann alternativ auch aus einer doppelt ungesättigten Fettsäure durch Reduktion mittels einer 2,4-dienoyl-CoA-Reduktase, bei *E. coli* FadH, gebildet werden. Ein multifunktionelles Enzym, die Enoyl-CoA-Hydratase/3-Hydroxyacyl-CoA-Dehydrogenase, bei *E. coli* FadB, katalysiert anschließend die Hydratisierung unter Bildung des sekundären Alkohols und dessen anschließende Oxidation zum Keton. Im letzten Schritt katalysiert eine 3-Ketoacyl-CoA-Thiolase, im Falle von *E. coli* FadA, die Spaltung des Ketoacyl-CoA mit dem Ergebnis, dass Acetyl-CoA und ein im Vergleich zum Ausgangsmolekül um zwei Kohlenstoffatome verkürzter CoA-Ester der Fettsäure freigesetzt werden. Sofern es sich nicht ebenfalls um Acetyl-CoA handelt, kann letzterer erneut in den β-Oxidationszyklus eingespeist und unter Oxidation verkürzt werden. An der Regulation der β-Oxidation von Fettsäuren ist auch FadR beteiligt, ein Regulator des Fad-Operons, der die für den Abbau von Fettsäuren erforderlichen Gene umfasst, ohne dass FadR eine Reaktion der β-Oxidation katalysieren würde. In einer bevorzugten Ausführungsform wird unter dem Begriff "Enzym, das eine der Reaktionen der β-Oxidation von Fettsäuren katalysiert" ein jegliches Enzym verstanden, das direkt mit dem Fettsäuresubstrat oder einem auf dem Weg zum Acetyl-CoA daraus entstehenden Molekül wechselwirkt, bevorzugt es als Substrat erkennt, und seine Umwandlung zu einem auf diesem Abbauweg näher am Acetyl-CoA liegenden Stoffwechselprodukt katalysiert, bevorzugt einschließlich des Fettsäureimporters, der die Aufnahme der Fettsäure in die Zelle bewerkstelligt. Beispielsweise zählt zu diesen Enzymen nach der vorangegangenen Definition die Acyl-CoA-Dehydrogenase, da sie mit dem Fettsäure-CoA-Ester wechselwirkt und dessen Umwandlung zum Enyol-CoA katalysiert, das auf dem Stoffwechselweg der β-Oxidation näher am Acetyl-CoA liegt als der Fettsäure-CoA-Ester. In einer besonders bevorzugten Ausführungsform wird unter dem Begriff "Enzym, das eine der Reaktionen der β-Oxidation von Fettsäuren katalysiert", wie hierin verwendet, jedes Enzym aus der Gruppe verstanden, die die Genprodukte FadA, FadB, FadD, FadL und FadE aus *E. coli* und/oder deren Varianten oder Homologa aus anderen Organismen umfasst. Die Genprodukte FadA, FadB, FadD, FadL und FadE aus *E. coli* ebenso wie Varianten und Homologa aus zahlreichen weiteren biotechnologisch nutzbaren Organismen und ihre Nukleinsäure- und Polypeptidssequenzen sind im Stand der Technik beschrieben, beispielsweise FadA unter Zugangsnummer AP009048.1, FadB unter Zugangsnummer BAE77457.1, FadD unter Zugangsnummer BAA15609.1, und FadE unter Zugangsnummer BAA77891.2. Der Stand der Technik offenbart zahlreiche Tests, die speziell für die Messung der Aktivität von Enzymen geeignet sind, die eine der Reaktionen der β-Oxidation von Fettsäuren katalysieren, beispielsweise in K Kameda & W D Nunn (1981) J. Biol. Chem. 256, 5702-5707, Hi Marrakchi, W E DeWolf, C Quinn, J West, B J Polizzi, C Y So et al. (2003) Biochem. J. 370, 1055-1062, Lobo et al. (2001) und X Yu, T Liu, F Zhu, and C Khosla (2011) PNAS, elektronische Veröffentlichung vor Drucklegung*.*

Für die Effektivität des erfindungsgemäßen Ganzzellkatalysators ist es von Vorteil, wenn das umzusetzende Substrat, bevorzugt die Carbonsäure oder Dicarbonsäure oder ein Monoester davon, leicht mit den erfindungsgemäß erforderlichen Enzymen, die im Inneren des Ganzzellkatalysators lokalisiert sind, in Kontakt treten kann. Entscheidend ist daher, dass das Substrat ins Innere der Zelle gelangen kann. Um dies zu erleichtern, ist bevorzugt, dass der Ganzzellkatalysator einen Fettsäureimporter, im Falle eines bakteriellen, insbesondere Gram-negativen Ganzzellkatalysators, besonders bevorzugt den Fettsäureimporter FadL (Datenbankcode: BAA16205.1) oder eine Variante exprimiert, bevorzugt in einer Konzentration und mit einer Aktivität, die gegenüber der Aktivität des Wildtyps des entsprechenden Ganzzellkatalysators erhöht ist. Der Erhöhung der Aktivität eines Polypeptids gegenüber dem Wildtyp der Zelle kann über verschiedene, dem Fachmann routinemäßig zugängliche Wege erreicht werden, beispielsweise den Einbau zusätzlicher, funktionell mit einem Promotor verbundener Kopien der für das Polypeptid kodierenden Nukleotidsequenz oder den Austausch des natürlichen gegen einen stärkeren Promotor.

Es hat sich gezeigt, dass die Amine und Diamine erfindungsgemäß in höherer Ausbeute und Reinheit produziert werden, wenn der Hintergrund an im Ganzzellkatalysator endogen exprimierten Enzymen derart optimiert ist, dass die Aktivität von endogenen Enzymen verringert oder ausgeschaltet ist, die Edukte, Zwischenprodukte oder Produkte des erfindungsgemäßen Verfahrens oder unter Verwendung der erfindungsgemäßen Zelle, bevorzugt Methylester von ω-Aminocarbonsäuren, ω-Hydroxycarbonsäuren, ω-Oxocarbonsäuren und Dicarbonsäuren, auf Stoffwechselwegen abbauen oder anderweitig modifizieren, die von der Entstehung des erwünschten Produktes wegführen. Vor diesem Hintergrund kann es vorteilhaft sein, wenn es sich bei dem erfindungsgemäßen Ganzzellkatalysator um eine Zelle handelt, die ihrem Wildtyp gegenüber eine verringerte Aktivität der Esterase BioH [Datenbankcode YP_492020.1] oder einer Variante davon aufweist. Derartige Zellen mit verringerter BioH-Aktivität, ihre Herstellung und Tests zur Aktivitätsbestimmung sind in der Europäischen Patentanmeldung EP 12007663.3 beschrieben.

Für den Fall, dass als Carbonsäure, Dicarbonsäure oder Monoester davon Mischungen von Verbindungen eingesetzt werden, bei denen die endständigen Carboxygruppen in hohem Maße, bevorzugt zu wenigstens 50, 60, 70, 80, 90, 95 oder 99 Prozent in Form eines Esters vorliegt, beispielsweise aus Gründen der besseren Verfügbarkeit dieser Substrate oder der Toxizität freier Carbonsäuren oder Dicarbonsäuren, kann der Monoester durch teilweise oder vollständige Hydrolyse von vollständig veresterten Dicarbonsäuren bzw. die freie Carbonsäure durch teilweise oder vollständige Hydrolyse von vollständig veresterten Carbonsäuren bereitgestellt werden. Für diesen Fall es vorteilhaft sein, die Kapazität der Zelle zur Esterhydrolyse durch Überexpression einer geeigneten Esterase zu steigern. In einer bevorzugten Ausführungsform wird dazu die Aktivität der Esterhydrolase BioH oder einer Variante davon gegenüber dem Wildtyp des eingesetzten Ganzzellkatalysators erhöht, besonders bevorzugt durch Überexpression. Der entsprechende Monoester und/oder die unveresterte Carbonsäure oder Dicarbonsäure wird dann *in situ* durch Esterhydrolyse bereitgestellt. Die teilweise oder vollständige Hydrolyse einer vollständig veresterten Dicarbonsäure kann auch durch chemisch katalysierte Hydrolyse erfolgen, beispielsweise bei niedrigen pH-Werten.

Der erfindungsgemäße Ganzzellkatalysator kann in einem Verfahren zur Umsetzung von einer Carbonsäure oder Dicarbonsäure oder eines Monoesters davon zum entsprechenden Amin oder Diamin, bevorzugt eingesetzt werden, wobei es sich bei der Carbonsäure oder Dicarbonsäure oder dem Monoester davon um eine Verbindung der Formel (I)

R¹- A - COOR² (I)

handelt, wobei R¹ aus der Gruppe ausgewählt ist, die -H und COOR³ umfasst, wobei R² und R³ jeweils und unabhängig voneinander aus der Gruppe ausgewählt sind, die H, Methyl, Ethyl und Propyl umfasst, mit der Maßgabe, dass wenigstens einer der Reste R² und R³ H ist, wobei A eine unverzweigte, verzweigte, lineare, zyklische, substituierte oder unsubstituierte Kohlenwasserstoffgruppe mit wenigstens vier Kohlenstoffatomen darstellt. In einer bevorzugten Ausführungsform handelt es sich bei A um eine Struktur der Formel -(CH₂)ₙ⁻, wobei n bevorzugt 4, 5, 6, 7, 8, 9, 10, 11, 12, 13, 14, 15, 16, 17, 18, 19, 20, 21, 22, 23, 24, 25, 26, 27, 28, 29 oder 30 ist. In einer bevorzugtesten Ausführungsform handelt es sich bei der Carbonsäure oder Dicarbonsäure um Laurinsäure oder ω-Carboxylaurinsäure. In einer weiteren bevorzugtesten Ausführungsform handelt es sich bei der Carbonsäure um eine Carbonsäure der Formel CH₃-(CH₂)ₙ-COOH, wobei n bevorzugt 4, 5, 6, 7, 8, 9, 10, 11, 12, 13, 14, 15, 16, 17, 18, 19, 20, 21, 22, 23, 24, 25, 26, 27, 28, 29 oder 30 ist, bevorzugt um Hexansäure oder Decansäure. In einer weiteren bevorzugtesten Ausführungsform handelt es sich bei der Carbonsäure oder Dicarbonsäure um eine Dicarbonsäure der der Formel HOOC-(CH₂)ₙ-COOH, wobei n bevorzugt 4, 5, 6, 7, 8, 9, 10, 11, 12, 13, 14, 15, 16, 17, 18, 19, 20, 21, 22, 23, 24, 25, 26, 27, 28, 29 oder 30 ist, bevorzugt um ω-Carboxyhexansäure oder ω-Carboxydecansäure. In einer weiteren bevorzugtesten Ausführungsform handelt es sich bei der Carbonsäure oder Dicarbonsäure um ω-Carboxytetradecansäure. Entsprechend handelt es sich bei dem erfindungsgemäß hergestellten Amin bzw. Diamin bevorzugt um eine Verbindung der Formel CH₃-(CH₂)ₙ-NH₂ bzw. NH₂-(CH₂)ₙ-NH₂, wobei n jeweils bevorzugt 4, 5, 6, 7, 8, 9, 10, 11, 12, 13, 14, 15, 16, 17, 18, 19, 20, 21, 22, 23, 24, 25, 26, 27, 28, 29 oder 30 ist.

Mit Hinblick auf die Carbonsäure oder Dicarbonsäure oder den Monoester davon wie auf jede in dieser Anmeldung beschriebene chemische Verbindung gilt, dass die jeweilige angegebene Formel sämtliche Salze, protonierte oder deprotonierte der jeweiligen Verbindung umfasst. Beispielsweise umfasst die Laurinsäure nicht nur die protonierte Form, sondern auch das Salz Laureat mit sämtlichen Kationen, beispielsweise Natriumlaureat.

Das erfindungsgemäße Verfahren erfordert, dass die für das erfindungsgemäße Verfahren eingesetzten Enzyme, optional in Form des erfindungsgemäßen Ganzzellkatalysators bereitgestellt, mit der Carbonsäure oder Dicarbonsäure oder dem Monoester davon in einer wässrigen Lösung kontaktiert werden. In einer bevorzugten Ausführungsform wird unter dem Begriff "Kontaktieren", wie hierin verwendet, verstanden, dass das jeweilige Enzyme in unmittelbaren Kontakt mit seinem Substrat gelangt, insbesondere ohne dass physikalische Barrieren wie impermeable Membranen oder dergleichen zwischengeschaltet sind. Das Kontaktieren geschieht im einfachsten Fall dadurch, dass das Substrat zu einer wässrigen Lösung gegeben wird, in der sich das Enzym oder der Ganzzellkatalysator befindet.

Zur Ausführung der erfindungsgemäßen Lehre eignet sich eine Reaktionsmischung umfassend den erfindungsgemäßen Ganzzellkatalysator 8 in wässriger Lösung sowie eine Carbonsäure oder Dicarbonsäure oder einen Monoester davon der Formel (I), wobei R¹ aus der Gruppe ausgewählt ist, die -H COOR³ umfasst, wobei R² und R³ jeweils und unabhängig voneinander aus der Gruppe ausgewählt sind, die H, Methyl, Ethyl und Propyl umfasst, mit der Maßgabe, dass wenigstens einer der Reste R² und R³ H ist, wobei A eine unverzweigte, verzweigte, lineare, zyklische, substituierte oder unsubstituierte Kohlenwasserstoffgruppe mit wenigstens vier Kohlenstoff darstellt, bevorzugt die Formel - (CH₂)ₙ -, wobei n wenigstens 4, besonders bevorzugt wenigstens 10 ist. Die wässrige Lösung muss, beispielsweise mit Hinblick auf Zusammensetzung, pH-Wert und Temperatur, dabei derart beschaffen sein, dass sie die Lebensfähigkeit oder zumindest die katalytische Fähigkeit des Ganzzellkatalysators wenigstens zeitweilig unterstützt. Dem Fachmann sind zahlreiche als wässrige Lösung geeignete wässrige Kulturmedien bekannt, die für die Erhaltung oder Kultivierung von Zellen, insbesondere biotechnologisch bedeutsamer Zellen, geeignet sind. Darunter fallen gleichermaßen Vollmedien wie LB-Medien, Minimalmedien wie M9-Medien sowie Selektivmedien, beispielsweise solche, die eine hohe Salzkonzentration enthalten und daher nur das Wachstum halophiler oder zumindest halotoleranter Organismen ermöglichen. In einer bevorzugten Ausführungsform wird unter dem Begriff "wässriges Kulturmedium", wie hierin verwendet, ein Reaktionsmedium auf Wasserbasis verstanden, das mit Hinblick auf sämtliche relevante Faktoren, insbesondere pH-Wert, Salzgehalt und Temperatur, derart beschaffen ist, das es die Lebensfähigkeit darin enthaltener Zellen, bevorzugt Mikroorganismen, erhält oder fördert und sowohl wässriges Kulturmedium als auch hydrophobe organische Phase in flüssiger Form vorliegen. Die Temperaturansprüche verschiedener biotechnologisch bedeutsamer Zellen können mikrobiologischen und molekularbiologischen Lehrbüchern entnommen werden, z.B. Fuchs/Schlegl, 2008. In einer bevorzugten Ausführungsform liegt der pH-Wert des wässrigen Kulturmediums zum Zeitpunkt des Kontaktierens zwischen 4 bis 9, bevorzugter zwischen 4,5 bis 8,5, am bevorzugtesten zwischen 6,5 und 7,5. In einer weiteren bevorzugten Ausführungsform liegt die Temperatur zwischen 0 und 45 °C, bevorzugter zwischen 15 und 40 °C, am bevorzugtesten zwischen 20 und 37 °C. Die Reaktionsmischung ist typischerweise in einem Fermenter enthalten. Als Fermenter kann ein jedes Reaktionsgefäß fungieren, das sterilisiert, bevorzugt autoklaviert werden kann und die Anzucht des Ganzzellkatalysators, Belüftung und Steuerung der Reaktionsbedingungen, beispielsweise des Sauerstoffgehalts und der Temperatur, erlaubt.

In einer bevorzugten Ausführungsform umfasst die Reaktionsmischung zusätzlich zur wässrigen Lösung eine hydrophobe organische Phase. Diese kann ein organisches Lösungsmittel und/oder einen hydrophoben flüssigen Kationenaustauscher zur Entfernung der ω-Aminofettsäure aus der wässrigen Lösung umfassen. Geeignete Lösungsmittel und Kationenaustauscher sind in der EP11191520.3 beschrieben.

Die vorliegende Erfindung wird weiterhin durch die folgenden Figuren und nicht beschränkenden Beispiele veranschaulicht, denen weitere Merkmale, Ausführungsformen, Aspekte und Vorteile der vorliegenden Erfindung entnommen werden können.

## Patentansprüche

1. Ganzzellkatalysator, der eine rekombinante α-Dioxygenase oder die Kombination aus einer rekombinanten Fettsäurereduktase und aus einer die Fettsäurereduktase phosphopantheteinylierenden Phosphopantheteinyl-Transferase exprimiert, und der zusätzlich eine Transaminase exprimiert, wobei die Phosphopantheteinyl-Transferase und/oder Transaminase bevorzugt rekombinant ist.

2. Ganzzellkatalysator nach Anspruch 2, wobei der Ganzzellkatalysator zusätzlich eine Aminosäuredehydrogenase exprimiert, die bevorzugt rekombinant ist.

3. Ganzzellkatalysator nach einem der Ansprüche 1 bis 2, wobei der Ganzzellkatalysator zusätzlich eine Alkanhydroxylase exprimiert, die bevorzugt rekombinant ist.

4. Ganzzellkatalysator nach einem der Ansprüche 1 bis 3, wobei der Ganzzellkatalysator zusätzlich ein Polypeptid der AlkL-Familie exprimiert, das bevorzugt rekombinant ist.

5. Ganzzellkatalysator nach einem der Ansprüche 1 bis 4, der zusätzlich eine Alkoholdehydrogenase exprimiert, die bevorzugt rekombinant ist.

6. Ganzzellkatalysator nach einem der Ansprüche 1 bis 5, wobei die Aktivität wenigstens eines an der β-Oxidation beteiligten Enzyms gegenüber dem Wildtyp des Ganzzellkatalysators verringert ist.

7. Ganzzellkatalysator nach einem der Ansprüche 1 bis 6, wobei die Aktivität von BioH oder einer Variante davon gegenüber dem Wildtyp des Ganzzellkatalysators verringert oder erhöht ist.

8. Ganzzellkatalysator nach einem der Ansprüche 1 bis 7, wobei die Aktivität von FadL oder einer Variante davon gegenüber dem Wildtyp des Ganzzellkatalysators erhöht ist.

9. Verfahren zur Umsetzung einer Carbonsäure oder Dicarbonsäure oder eines Monoesters davon zu einem Amin oder Diamin, umfassend die Schritte
a) Bereitstellen einer Carbonsäure oder Dicarbonsäure oder eines Monoesters davon, für den Fall, dass es sich um eine Dicarbonsäure handelt, bevorzugt durch Kontaktieren einer Carbonsäure mit einer Alkanhydroxylase und/oder Alkoholdehydrogenase,
b) Kontaktieren des Carbonsäure oder Dicarbonsäure oder des Monoesters davon mit einer phosphopantheteinylierten Fettsäurereduktase oder einer α-Dioxygenase unter Bildung eines Aldehydproduktes, und
c) Kontaktieren des Aldehydproduktes aus Schritt a) mit einer Transaminase.

10. Verfahren nach Anspruch 9, wobei in Schritt c) eine Aminosäuredehydrogenase anwesend ist.

11. Verfahren nach einem der Ansprüche 9 bis 10, wobei wenigstens ein Enzym aus der Gruppe umfassend phosphopantheteinylierte Fettsäurereduktase, α-Dioxygenase, Transaminase, Aminosäuredehydrogenase und Alkanhydroxylase, bevorzugt alle verwendeten Enzyme aus dieser Gruppe, in Form eines Ganzzellkatalysators nach einem der Ansprüche 1 bis 8 bereitgestellt werden.

12. Verfahren nach einem der Ansprüche 9 bis 11, wobei es sich bei der Carbonsäure oder Dicarbonsäure oder dem Monoester davon um eine Verbindung der Formel (I)
R¹ - A - COOR² (I)
handelt, wobei R¹ aus der Gruppe ausgewählt ist, die -H und COOR³ umfasst,
wobei R² und R³ jeweils und unabhängig voneinander aus der Gruppe ausgewählt sind, die H, Methyl, Ethyl und Propyl umfasst,
mit der Maßgabe, dass wenigstens einer der Reste R² und R³ H ist,
wobei A eine unverzweigte, verzweigte, lineare, zyklische, substituierte oder unsubstituierte Kohlenwasserstoffgruppe mit wenigstens vier Kohlenstoff darstellt.

13. Verfahren nach einem der Ansprüche 9 bis 12, wobei A die Formel - (CH₂)ₙ - aufweist, wobei n wenigstens 4, bevorzugt wenigstens 10 ist.

14. Verwendung des Ganzzellkatalysators nach einem der Ansprüche 1 bis 8 oder des Verfahrens nach einem der Ansprüche 8 bis 12 zur Aminierung einer Carbonsäure oder Dicarbonsäure oder eines Monoesters davon.

15. Reaktionsmischung umfassend den Ganzzellkatalysator nach einem der Ansprüche 1 bis 8 in wässriger Lösung sowie eine Carbonsäure oder Dicarbonsäure oder einen Monoester davon der Formel (I)
R¹ - A - COOR² (I),
wobei R¹ aus der Gruppe ausgewählt ist, die -H und COOR³ umfasst,
wobei R² und R³ jeweils und unabhängig voneinander aus der Gruppe ausgewählt sind, die H, Methyl, Ethyl und Propyl umfasst,
mit der Maßgabe, dass wenigstens einer der Reste R² und R³ H ist,
wobei A eine unverzweigte, verzweigte, lineare, zyklische, substituierte oder unsubstituierte Kohlenwasserstoffgruppe mit wenigstens vier Kohlenstoff darstellt, bevorzugt die Formel - (CH₂)ₙ -, wobei n wenigstens 4, besonders bevorzugt wenigstens 10 ist.
